# EUROPEAN PATENT APPLICATION

(11) **EP 2 201 934 A1**
(43) Date of publication of application: **30.06.2010**
(21) Application number: 08172814.9
(22) Date of filing: 23.12.2008
(51) Int. Cl.: A61K 9/00, A61K 31/46, A61M 15/00

(54) **Tiotropium aerosol formulation products with improved chemical stability**

(71) Applicant: CHIESI FARMACEUTICI S.p.A., 43100 Parma (IT)
(72) Inventor: The designation of the inventor has not yet been filed
(74) Representative: Minoja, Fabrizio

(57) **Abstract**

The invention relates to a pressurized metered dose inhaler, comprising an aerosol can fitted with a metering valve provided with the sealing ring/s and/or gasket/s, which is/are in contact with the formulation, is/are made of a butyl or halo-butyl rubber, wherein the aerosol can contains a medicinal aerosol solution formulation comprising
a tiotropium salt,
a hydrofluorocarbon propellant,
one or more co-solvents, and
a mineral acid as a stabilizer for the active ingredient.

The invention also relates to the process for the preparation thereof, and to their use for the manufacturing of a medicament for the prevention and/or treatment of an inflammatory or obstructive airways disease such as asthma or chronic obstructive pulmonary disease (COPD).

## Description

### FIELD OF THE INVENTION

The invention relates to a pressurised metered dose inhaler (MDI) containing a stable pharmaceutical aerosol formulation wherein the active ingredient is in solution in one or more hydrofluoroalkane (HFA) propellants.

In particular, the present invention relates to a pressurized MDI containing a solution formulation suitable for aerosol administration comprising a tiotropium salt, and in particular tiotropium bromide, which is stable at room temperature for a pharmaceutically acceptable shelf-life.

The invention also relates to the process for the preparation thereof.

### BACKGROUND OF THE INVENTION

Quaternary ammonium salts acting as muscarinic receptor antagonists are currently used in therapy to induce bronchodilation for the treatment of respiratory diseases and in particular inflammatory or obstructive airway diseases such as asthma and chronic obstructive pulmonary disease (COPD).

The use of antimuscarinic drugs with a long-lasting effect is often desirable for treating chronic diseases. This ensures that the concentration of the active substance necessary for achieving the therapeutic effect is present in the lungs for a long period of time, without the need for the active substance to be administered repeatedly and too frequently.

In particular, it would be highly desirable to utilize antimuscarinic drugs which are therapeutically efficacious upon administration by inhalation once a day.

In order to fulfill such a requirement, antimuscarinic drugs shall exhibit good selectivity for M3 muscarinic receptors, and slow dissociation from them.

It has been reported that a tiotropium salt, and in particular its bromide salt, exhibits a very slow dissociation from M3 receptors. This behaviour is thought to account for its long lasting activity and therefore might provide a significant therapeutic benefit in the treatment of respiratory diseases such as asthma and COPD (chronic obstructive pulmonary disease), when administered by inhalation.

Antimuscarinic drugs are currently administered to the respiratory tract through:
(a) metered dose inhalers (pMDIs), which use a chlorofluorocarbon (CFC) or the most environmental safe hydrofluoroalkane (HFA) propellant or propellant mixtures, pressurised formulation to deliver the active ingredient as an aerosol;
(b) dry powder inhalers (DPIs) which deliver the micronised active ingredient as a dry powder formulation in presence of a suitable carrier; or
(c) jet or ultrasonic nebulizers known from the prior art or by inhaler devices known as soft-mist nebulizers which deliver propellant-free inhalable formulations in form of solutions or suspensions in an aqueous, alcoholic or hydroalcoholic medium.

Tiotropium bromide is currently marketed only as a DPI formulation or as a propellant-free solution for inhalation but not as pressurised MDI formulation.

To increase the compliance of patients affected by inflammatory respiratory diseases such as asthma and COPD, the need exists to prepare an inhalable formulation of tiotropium bromide to be administered by a pressurised MDI.

Formulations for pressurised MDIs can be solutions or suspensions. Solution formulations offer the advantage of being homogeneous in nature with the medicament and excipient completely dissolved in the propellant vehicle.

Solution formulations also obviate physical stability problems associated with suspension formulations and thus assure more consistent uniform dosage administration while also eliminating the need for suspending agents such as surfactants.

However solution formulations may suffer from chemical stability problems of the active ingredient in the formulation vehicle, generally comprising the propellant and a co-solvent, when the active ingredient is highly susceptible to chemical degradation.

The possibility to administer tiotropium bromide from a HFA pressurised MDI solution formulation is generally disclosed in EP 673240 which suggests the addition of inorganic or organic acids to a HFA aerosol formulation in order to provide for the stabilization of the medicament.

Corrosion resistant high grade steel cans for storing a HFA aerosol formulation which contains ethanol, various active substances including tiotropium bromide and, optionally, citric acid or a mineral acid such as hydrochloric acid are disclosed in EP 1198395.

Stabilized aerosol solution formulations comprising a tiotropium salt, a HFA propellant, a co-solvent, and an inorganic or organic acid, **characterized in that** the concentration of the acid is in a range that corresponds to a pH range of 2.5-4.5 in an aqueous solution are disclosed in WO 2004/054580. However no evidence that the problem of chemical stability has been solved is given there.

MDIs generally comprise a pressure resistant aerosol can filled with a product such as a drug dissolved in a liquefied propellant or micronized particles suspended in a liquefied propellant where the container is fitted with a metering valve. Actuation of the metering valve allows a small portion of the aerosol product to be released whereby the pressure of the liquefied propellant carries the dissolved or micronized drug particles out of the container to the patient. The valve actuator is used to direct the aerosol spray into the patient's airways.

Generally, the valve includes rubber valve seals (diaphragm, gasket or sealing rings) intended to allow reciprocal movement of the valve stem while preventing leakage of propellant from the container.

Said rubber valve seals are commonly made of elastomeric material based on the traditional technology of vulcanising a synthetic or natural rubber polymer.

Among the elastomeric materials for the gaskets of metered dose inhalers ethylene-propylene-diene monomer (EPDM) rubber, disclosed in EP 708805, has been demonstrated as one of the most commonly used in MDIs pressurised by hydrofluorocarbon (HFA or HFC) propellants in view of their stability to dimensional change and to avoid propellant leakage or sticking of the valve stem.

It has been now surprisingly found that a stable tiotropium HFA aerosol solution formulation may be obtained using a pressurized metered dose inhaler, comprising an aerosol can equipped with a metering valve provided with at least one of the sealing rings and/or gaskets, which may be in contact with the formulation, made of a butyl rubber of specific composition in addition to the use of an inorganic acid in the formulation.

### SUMMARY OF THE INVENTION

In one aspect the present invention provides a pressurized metered dose inhaler, comprising an aerosol can fitted with a metering valve comprising sealing ring/s and/or gasket/s, which is/are in contact with the formulation, made of a butyl or halo-butyl rubber wherein the aerosol can contains a medicinal aerosol solution formulation comprising
a tiotropium salt,
a hydrofluorocarbon propellant,
one or more co-solvents, and
a mineral acid.

According to one embodiment said tiotropium salt is tiotropium bromide.

According to one embodiment the mineral acid is phosphoric acid.

In one embodiment said pressurized metered dose inhaler, comprises an aerosol can fitted with a metering valve comprising sealing ring/s and/or gasket/s, which is/are in contact with the formulation, made of a butyl or halo-butyl rubber which is a vulcanisate of an elastomeric composition of a butyl or halo-butyl rubber, a cross-linking agent for the butyl or halo-butyl rubber, and an accelerator for the cross-linking agent, in particular the accelerator may include a polysulphide compound derived from a substituted dithiocarbonic acid or derivative thereof.

According to one embodiment part or all of the internal surfaces of said aerosol can consists of conventional aluminium, anodised aluminium or is lined with an inert organic coating which is a perfluoroalkoxy alkane, a perfluoroalkoxyalkylene, a perfluoroalkylene such as polytetrafluoroethylene, epoxy-phenol resin or fluorinated ethylene-propylene, polyether sulfone or blends thereof.

In a further aspect, the present invention comprises the use of the pressurised metered dose inhaler described above for the manufacturing of a medicament for the prevention and/or treatment of an inflammatory or obstructive airways disease such as asthma or chronic obstructive pulmonary disease (COPD).

In still a further aspect, the present invention comprises a method of preventing and/or treating an inflammatory or obstructive airways disease such as asthma or chronic obstructive pulmonary disease (COPD), which comprises administration by inhalation of an effective amount of one of the medicinal aerosol formulation contained in the pressurised metered dose inhaler described above.

According to a further aspect, the present invention provides a process for filling a pressurized metered dose inhaler, comprising an aerosol can fitted with a metering valve provided with sealing rings and/or gaskets made of a butyl or halo-butyl rubber with a medicinal aerosol solution formulation comprising a tiotropium salt, a hydrofluorocarbon propellant, one or more co-solvents, and a mineral acid, wherein said process comprises filling said can with a solution of a tiotropium salt in one or more co-solvent, adding a pre-determined amount of a mineral acid, further adding a propellant comprising a hydrofluoroalkane (HFA) to said solution and crimping with the metering valve. In alternative the propellant may be added through the valve in the final step after having crimped the valve on the can containing the mixture of a tiotropium salt, the co-solvent and the acid.

### DEFINITIONS

An "elastomer" is a polymer with the property of elasticity. The term is often used interchangeably with the term "rubber". However an "elastomer" has a synthetic origin and it is also named "synthetic rubber" while if it has a natural origin it is named "natural rubber". The primary use of elastomers is for seals, adhesives and molded flexible parts.

"Butyl rubber" is a synthetic rubber and it is a copolymer made from isobutylene and a small amount of a diolefin, such as isoprene (2-methylbuta-1,3-diene) and it is well known in the art as it was first developed about seventy years ago by researchers of Standard Oil's.

Halogenated butyl rubbers (halo-butyl) were later developed in their chlorinated ("chloro-butyl") or brominated ("bromo-butyl") variants.

The terms "valve seals", "diaphragm", "gaskets" or "sealing rings" are herein used as synonyms.

The term "medicinal aerosol solution formulation" means a pharmaceutical formulation of a medicament suitable for aerosol administration wherein the active ingredient and the excipients are completely dissolved.

The terms "active drug", "active ingredient", "active", "active compound", "active substance", and "therapeutic agent" are used as synonymous.

As used herein, the expression "% w/w" mean the weight percentage of the component with respect to the total weight of the composition.

For "actuation" it is meant the release of the aerosol formulation from the device by a single activation of the inhaler (e.g. mechanical, inhalation or breath activated).

As used herein the term "mass median diameter" means the diameter of 50 percent by weight of the particles.

As used herein, the term "co-solvent" means a substance having a higher polarity than that of the propellant, and a vapour pressure at 25°C not less than 3 kPa, more preferably not less than 5 kPa.

As used herein, the expression "formulation chemically stable" means a formulation wherein the stability and the shelf-life of the active ingredient meet the requirements of the ICH Guideline Q1A referring to "Stability Testing of new Active Substances (and Medicinal Products)".

### BRIEF DESCRIPTION OF THE DRAWINGS

Figure 1 shows a conventional metered dose inhaler of the prior art.
Figure 2a shows a metering valve for inverted use.
Figure 2b shows a metering valve for upright use.

### DETAILED DESCRIPTION OF THE INVENTION

The present invention provides a pressurized metered dose inhaler, comprising an aerosol can fitted with a metering valve provided with at least one of the sealing rings and/or gaskets, which may be in contact with the formulation, made of a butyl rubber, or a halo-butyl rubber wherein the aerosol can contains a medicinal aerosol solution formulation comprising
a tiotropium salt,
a hydrofluorocarbon propellant,
one or more co-solvents, and
a mineral acid.

The invention further provides a pressurized metered dose inhaler, comprising an aerosol can fitted with a metering valve provided with at least one of the sealing rings and/or gaskets, which may be in contact with the formulation, made of a vulcanisate of an elastomeric composition of a butyl rubber or halo-butyl rubber, a cross-linking agent for the butyl or halo-butyl rubber and an accelerator for the cross-linking agent, wherein the accelerator includes a polysulphide compound derived from a substituted dithiocarbonic acid or derivative thereof wherein the aerosol can contains a medicinal aerosol solution formulation comprising
a tiotropium salt,
a hydrofluorocarbon propellant,
one or more co-solvents, and
a mineral acid.

Examples of suitable mineral acids are hydrochloric, phosphoric, nitric and sulphuric acid in form of concentrated or diluted aqueous solutions.

The pressurised metered dose inhaler according to the invention comprises an aerosol can fitted with a metering valve whose actuation allows the delivery, as an aerosol, of a full single therapeutically effective dose of the active ingredient.

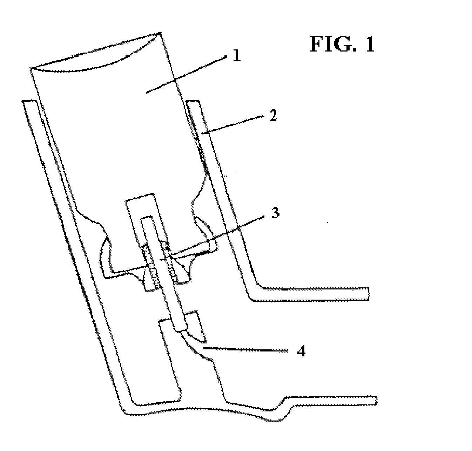

Figure 1 shows a conventional metered dose inhaler comprising a can (1), an actuator (2), a metering valve (3) and an actuator orifice (4).

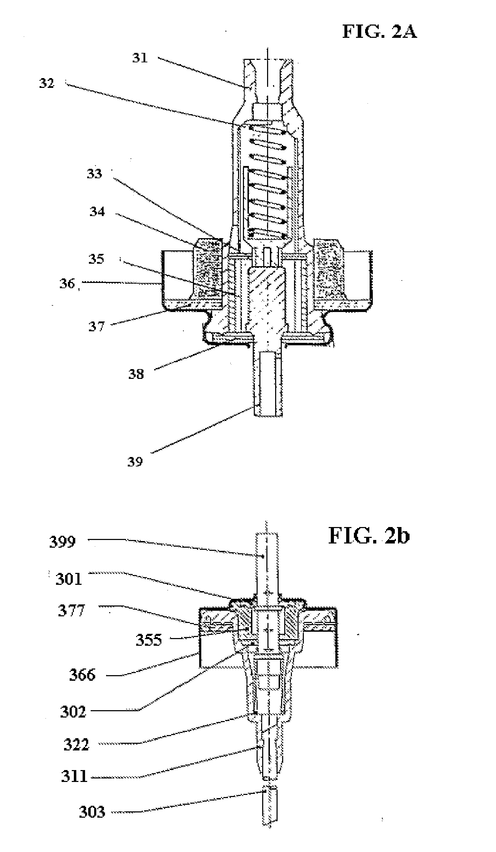

The metering valve should deliver accurately a measured amount of product that should be reproducible not only for each dose delivered from the same package, but from package to package. Two basic types of metering valves are available, one for inverted use and the other for upright use. Generally, valves for upright use contain a thin capillary dip tube (303) and are used with solution type aerosols. On the other hand, suspension or dispersion aerosols use a valve for inverted use, which does not contain a dip tube. Figures 2a and 2b show conventional valve designs for upright (Fig. 2b) and inverted use (Fig. 2a).

Generally the metering valve assembly comprises a ferrule (36; 366) having an aperture formed therein, a body molding (34; 311) attached to the ferrule which houses the metering chamber (35; 355), a stem (39; 399), an inner-seal (33; 302) and an outer-seal (38; 301) around the metering chamber, a spring (32; 322), and a sealing gasket (37; 377) to prevent leakage of propellant through the valve.

In general terms the valve seals, especially the sealing gasket (37; 377), as well as the inner-seal (33; 202) and the outer-seal (5), shall preferably be manufactured of a material which is inert to and resists extraction into the contents of the formulation, especially when the contents include ethanol.

It is to be understood that the present invention is not limited to the kind of valves represented in the figures, but any valve provided with at least one of the sealing rings and/or gaskets, which may be in contact with the formulation, made of an elastomeric composition of a butyl or halo-butyl rubber may be included in the scope of the invention.

The metering valve is provided with sealing rings and/or gaskets made of a butyl rubber which may be a conventional butyl, halo-butyl rubber or a particular vulcanisate of an elastomeric composition of a butyl rubber, a cross-linking agent for the butyl rubber, and an accelerator for the cross-linking agent, wherein the accelerator includes a polysulphide compound derived from a substituted dithiocarbonic acid or derivative thereof which is a material of the type described in WO 03/078538.

A conventional butyl rubber is a copolymer made from isobutylene and a small amount of a diolefin, such as isoprene (2-methylbuta-1,3-diene). Typically, according to the present invention, butyl rubber comprises approximately 97% isobutylene and approximately 3% isoprene, and it may be polymerised using an aluminium chloride catalyst.

For the purposes of the present invention, particularly preferred are the halogenated butyl rubbers, chlorobutyl or bromobutyl rubbers of the above-referenced composition (approx. 97% isobutylene and approx. 3% isoprene) among which bromobutyl rubbers are the most preferred.

The cross-linking agent (also known as the curing agent) provides or facilitates network formation to result in a three-dimensional polymer network structure. The cross-linking agent will typically comprise sulphur or a sulphur-containing compound. The cross-linking agent is preferably substantially free of any peroxide curing agents such as dicumyl peroxide.

The polysulphide compound used as the accelerator is preferably derived from a substituted xanthic acid or a derivative thereof, preferably of the type ROC(S)SH, in which R is typically an C1-C6 alkyl radical. The substituted group in the polysulphide compound typically comprises an isopropyl group.

The polysulphide compound preferably comprises three or more bridging sulphur atoms, more preferably 3, 4 or 5 bridging sulphur atoms.

The polysulphide compound is preferably substantially free of nitrogen, phosphorous and metallic elements.

Advantageously, the polysulphide compound comprises or consists of diisopropyl xanthogen polysulphide.

The elastomeric composition for preparing the vulcanisate typically comprises up to 3% by weight of the accelerator based on the total weight of the accelerator and butyl rubber in the composition, more typically up to 1.5% by weight of the accelerator based on the total weight of the accelerator and butyl rubber in the composition, still more typically up to 1% by weight of the accelerator based on a total weight of the accelerator and butyl rubber.

The weight ratio of the accelerator to the cross-linking agent in the elastomeric composition is preferably in the range of from 1:1 to 3:1, more preferably from 1:1 to 2:1.

The sealing rings and/or gaskets may further include a filler, preferably a mineral filler, a process aid, preferably a low molecular weight polyethylene and further auxiliary ingredients as defined on page 9, line 28 to page 10, line 26 of WO 03/078538.

The sealing rings and/or gaskets of the metering valve may be provided as a separate component or may be formed integrally with the valve.

Preferably, the rubbers are extracted with a suitable pharmaceutically acceptable solvent, preferably warm ethanol, before their assembling in the metered dose inhaler. In general, solvents which are pharmaceutically acceptable and endowed with adequate capacity of extraction of oxides and peroxides can be utilised.

Part or all of the internal surfaces of said aerosol may consists of aluminium, anodised aluminium or is lined with an inert organic coating. Examples of preferred coatings are epoxy-phenol resins, fluorocarbon polymers which can be constituted of multiples of the following monomeric units: perfluoroalkoxyalkanes (PFA), perfluoroalkoxyalkylenes, perfluoroalkylenes such as tetrafluoroethylene (PTFE or Teflon), or fluorinated ethylene-propylene (FEP), ethylene tetrafluoroethylene (ETFE), vinyldienefluoride (PVDF) and chlorinated ethylene tetrafluoroethylene. Additionally, in other embodiments, cans having inner surfaces coated with non-fluorocarbon polymers such as polyamide, polyimide, polyamideimide, polyphenylene sulfide and polyether sulfone or their combinations and blends with fluorocarbon polymers or copolymers thereof may also be employed.

Cans having part or all of the internal surfaces in conventional aluminium or lined with Teflon or with a blend of fluorinated ethylene-propylene and polyethersulfone (PES) may preferably be used.

The formulation shall be actuated by a metering valve able of delivering a volume of between 50 µl and 100 µl, e.g. 50 µl or 63 µl.

100 µl is also suitable.

Advantageously the MDI device filled with the formulation may be fitted with a dose counter. For instance, said types of devices are described in the pending application n. EP 1758631 and EP 1787668.

The aerosol can contains a medicinal aerosol solution formulation comprising a pharmaceutically acceptable salt of tiotropium of formula 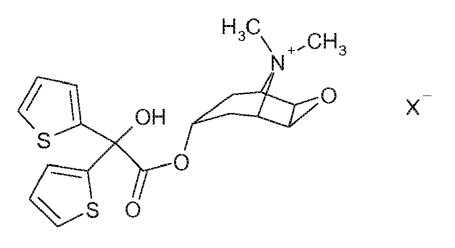 wherein X⁻ is a pharmaceutically acceptable anion, preferably selected from the group consisting of chloride, bromide, iodide, sulfate, phosphate, methanesulfonate, nitrate, maleate, acetate, citrate, fumarate, tartrate, oxalate, succinate, benzoate, and p-toluenesulfonate.

Tiotropium is preferably used in the form of its bromide salt and, depending on the choice of reaction conditions and solvents, obtainable in different crystalline modifications. Most preferred according to the invention are those formulations, that contain tiotropium anhydrous or in form of the tiotropium bromide monohydrate as disclosed in WO 02/30928.

Any pressure-liquefied propellant may be used, preferably a hydrofluoroalkane (HFA) propellant. Examples of HFA propellants include 1, 1, 1, 2-tetrafluoroethane (HFA 134a) and 1, 1, 1, 2, 3, 3, 3-heptafluoro-n-propane (HFA 227) and mixtures thereof.

The preferred propellant is 1, 1, 1, 2-tetrafluoroethane (HFA 134a).

The aerosol formulation according to the invention comprises a tiotropium salt of formula and in particular tiotropium bromide in an amount such that, in case of administration by inhalation from inhalers, the full single therapeutically effective dose (hereinafter simply indicated as the single dose) is advantageously comprised between about 1 µg and about 40 µg, preferably between about 2 µg and about 40 µg.

Said dose will depend on the kind and the severity of the disease and the conditions (weight, sex, age) of the patient and will be administered one or more times a day, preferably once a day.

The single dose may be delivered in one or two or more actuations (shots) of the inhaler wherein the pharmaceutical composition is contained. For example, a 10 µg single dose may be administered in one shot of 10 µg or as two shots of 5 µg dose.

In one embodiment, the single dose of a pharmaceutical composition comprising tiotropium bromide is comprised between 1 µg and 40 µg, preferably between 2 µg and 30 µg and more preferably between 10 µg and 20 µg.

The amount of a tiotropium salt in the composition can be calculated analogously if, instead of tiotropium bromide, another salt is used.

The medicinal aerosol formulations of the invention include a co-solvent having a higher polarity than that of the propellant and may include one or more additional co-solvent to solubilize the active ingredient in the propellant. Advantageously the co-solvent is selected from the group of lower branched or linear alkyl (C₁-C₄) alcohols such as ethanol and isopropyl alcohol.

Preferably the co-solvent is ethanol (95%), anhydrous ethanol but water may also be present in the formulation in order to favourably affect the solubility of tiotropium bromide.

The co-solvent will be present in an amount suitable to solubilize the active ingredient in the propellant in a concentration comprised between 6% and 30%, preferably between 8% and 25%, more preferably between 10% and 20%, even more preferably at 15% by weight based on the total weight of the formulation.

Small amounts of water between 0 and 5%, preferably between 1% and 4%, more preferably between 1% and 3%, even more preferably at 2% by weight based on the total weight of the formulation may be present.

In said formulations, depending on the volume of the metering valve to be used, tiotropium bromide may be present in a concentration comprised between 0.001 and 0.2% (w/w), preferably between 0.01 and 0.08% (w/w), even more preferably between 0.02 and 0.04% (w/w), most preferably at 0.028% by weight based on the total weight of the formulation.

The formulation of the invention comprises in addition a strong mineral acid selected from hydrochloric, phosphoric, nitric and sulphuric acid as stabilizer.

Hydrochloric acid (0.02 M) or phosphoric acid (15 M) are the preferred acids for the stabilization of tiotropium bromide. Phosphoric acid (15 M) is particularly preferred.

Phosphoric acid (15 M, corresponding to 85% w/w) is contained in the medicinal aerosol formulation in an amount between 0.0004 and 0.040% w/w, preferably between 0.0008 and 0.03% w/w, more preferably between 0.001 and 0.02% w/w, even more preferably between 0.002 and 0.01% w/w, based on the total weight of the formulation.

Alternatively hydrochloric acid (0.02M) is contained in the medicinal aerosol formulation in an amount between 0.0003 and 0.15% w/w, preferably between 0.007 and 0.1 % w/w, more preferably between 0.014 and 0.09% w/w, even more preferably between 0.03 and 0.08% w/w, based on the total weight of the formulation.

Conventional bulk manufacturing methods and machinery well known to those skilled in the art of pharmaceutical aerosol manufacture may be employed for the preparation of large scale batches for the commercial production of filled cans.

The process for filling a pressurized metered dose inhaler, comprising an aerosol can fitted with a metering valve provided with sealing rings and/or gaskets according to the invention comprises filling said can with a solution of a tiotropium salt in one or more co-solvent, adding a pre-determined amount of a mineral acid, further adding a propellant comprising a hydrofluoroalkane (HFA) to said solution under conditions which are sufficiently cold that the formulation does not vaporize and crimping with the metering valve.

In alternative the propellant may be added through the valve in the final step after having crimped the valve on the can containing the mixture of a tiotropium salt, the co-solvent and the acid.

Preferably, the processes are carried out an in inert atmosphere, for instance by insufflating nitrogen, in order to avoid the uptake of humidity from the air.

Administration of the formulations of the invention may be indicated for the prevention and/or treatment of mild, moderate or severe acute or chronic symptoms or for prophylactic treatment of respiratory diseases such as asthma and chronic obstructive pulmonary disease (COPD). Other respiratory disorders characterized by obstruction of the peripheral airways as a result of inflammation and presence of mucus such as chronic obstructive bronchiolitis and chronic bronchitis may also benefit by this kind of formulation.

The invention is better illustrated by the following examples.

### Example 1

Aerosol solution formulations according to the invention are prepared according to the process reported above in the description.

The composition of each formulation is reported in Table 1.

**Table 1**

| *Formulation* | *Amounts* | | |
|---|---|---|---|
| | Per unit | | Nominal dose |
| **(a)** | mg | % (w/w) | µg |
| Tiotropium bromide | 2.8 | 0.028 | 20 |
| Ethanol | 1500.0 | 15.000 | |
| Water | 200.0 | 2.000 | |
| HCI 0.02M | 7.5 | 0.075 | |
| HFA 134a | 8289.7 | 82.897 | |

| **(b)** | mg | % (w/w) | µg |
|---|---|---|---|
| Tiotropium bromide | 2.8 | 0.028 | 20 |
| Anhydrous Ethanol | 1500.0 | 15.000 | |
| Water | 200.0 | 2.000 | |
| HCI 0.02M | 7.5 | 0.075 | |
| HFA 134a | 8289.7 | 82.897 | |

| **(b)** | | | µg |
|---|---|---|---|
| Tiotropium bromide | 2.8 | 0.028 | 20 |
| Anhydrous Ethanol | 1500.0 | 15.000 | |
| Water | 200.0 | 2.000 | |
| Phosphoric Acid 15M | 0.2 | 0.002 | |
| HFA 134a | 8297.0 | 82.970 | |

| **(c)** | | | µg |
|---|---|---|---|
| Tiotropium bromide | 2.8 | 0.028 | 20 |
| Anhydrous Ethanol | 1500.0 | 15.000 | |
| Water | 200.0 | 2.000 | |
| Phosphoric Acid 15M | 1.0 | 0.010 | |
| HFA 134a | 8296.2 | 82.962 | |

### Example 2

The formulation (a) of Example 1 is filled in conventional aluminium cans coated with an inert coating comprising a fluorinated and non-fluorinated polymeric blend (FEP-PES) and fitted with a metering valve having a 63 µl metering chamber and conventional EPDM sealing gaskets. Different cans are stored in upright and inverted position up to 3 months at 40°C.

After three months the percent amount of tiotropium bromide both in upright and inverted cans is 96 and 24% respectively. The results are obtained as a mean of two cans.

The tiotropium bromide content in analogous cans, fitted with the same valves, containing a corresponding formulation but without hydrochloric acid, stored at the same temperature for 3 months in upright and inverted cans was 91 and 66% respectively.

### Example 3

The formulation (b) of Example 1 is filled in conventional aluminium cans coated by an inert coating comprising a fluorinated and non-fluorinated polymeric blend (FEP-PES) and fitted with a metering valve having a 63 µl metering chamber and butyl rubber sealing gaskets according to the invention. The cans are stored in inverted position up to 1 months at 40°C.

After one month the percent amount of tiotropium bromide is 98%. The result is a mean of two cans.

The same formulation (b) stored upright in the same kind of cans fitted with a metering valve having conventional EPDM sealing gaskets stored 1 month in upright position (no interaction with the valve) at 40°C gives a tiotropium bromide content of 98%. The result is a mean of two cans.

The same formulations (b) stored upright in conventional aluminium cans or anodised aluminium cans fitted with a metering valve having conventional EPDM sealing gaskets stored 1 month in upright position at 40°C give a very low tiotropium bromide content (15 and 26% respectively). The result is a mean of two cans.

Therefore the problem of stability of tiotropium bromide HFA solution formulation is not resolved as in the prior art by simply adding an acid to the formulation but depends from the kind of the can and of the metering valve used whose interaction with the stored formulation may provoke high degradation of the active ingredient.

### Example 4

The formulation (c) of Example 1 (containing 0.002% w/w phosphoric acid 15M) is filled in conventional aluminium cans coated by an inert coating comprising a fluorinated and non-fluorinated polymeric blend (FEP-PES) and fitted with a metering valve having a 63 µl metering chamber and conventional EPDM sealing gaskets. The cans are stored in upright position up to 3 months at 40°C.

After 3 months the percent amount of tiotropium bromide is 93%. The result is a mean of two cans.

The formulation (d) of Example 1 (containing 0.01% w/w phosphoric acid 15M) is filled in conventional aluminium cans coated by an inert coating comprising a fluorinated polymeric mixture and fitted with a metering valve having a 63 µl metering chamber and conventional EPDM sealing gaskets. The cans are stored in upright position up to 3 months at 40°C.

After 3 months the percent amount of tiotropium bromide is 97%. The result is a mean of two cans.

Therefore tiotropium bromide formulations with 0.01 % w/w phosphoric acid 15M are more stable (tiotropium residual amount after 3 months 97%) than formulations with 0.002% w/w phosphoric acid 15M (tiotropium residual amount after 3 months 93%) or corresponding formulations with 0.075% w/w hydrochloric acid 0.02M (tiotropium residual amount after 3 months 91%) when the cans are stored upright.

### Example 5

The formulation (d) of Example 1 (containing 0.01% w/w phosphoric acid 15M) is filled in three different can types (conventional aluminium coated by an inert coating comprising a fluorinated polymeric mixture; conventional aluminium; anodised aluminium), each can type is fitted with metering valves having a 63 µl metering chamber and conventional EPDM sealing gaskets or butyl rubber sealing gaskets according to the invention. The cans are stored in both orientation (upright and inverted) up to 6 months at 40°C.

At each time-point (1, 3 and 6 months) the residual content of tiotropium bromide (in mg) content is determined by HPLC and the residual percentage of the active ingredient with respect to time 0 is reported as the mean value from two cans.

The results are reported in Table 2.

**Table 2**

| **Can Type** | **Metering Valve Gaskets Types** | | **Time-point (months)** | | | **Time-point (months)** | | **Time-point (months)** | |
|---|---|---|---|---|---|---|---|---|---|
| | | **Orientation** | **0** | **1** | | **3** | | **6** | |
| | | | **mg** | **mg** | **%** | **mg** | **%** | **mg** | **%** |
| Coated Cans | EPDM | **UP** | 2.67 | 2.66 | 100 | 2.69 | 100 | 2.56 | 96 |
| | | **UP** | 2.68 | 2.68 | | 2.68 | | 2.60 | |
| | | **INV** | 2.69 | 2.65 | 98 | 2.50 | 92 | 0.00 | 0 |
| | | **INV** | N/A | 2.59 | | 2.41 | | 0.00 | |
| Conventional Aluminium Cans | EPDM | **UP** | N/A | 1.91 | 68 | 2.61 | 97 | 1.21 | 66 |
| | | **UP** | N/A | 1.75 | | 2.61 | | 2.30 | |
| | | **INV** | N/A | 2.66 | 98 | 2.54 | 91 | 1.71 | 69 |
| | | **INV** | N/A | 2.62 | | 2.33 | | 1.98 | |
| Anodised Aluminium Cans | EPDM | **UP** | N/A | 2.59 | 94 | 0.00 | 19 | 0.00 | 0 |
| | | **UP** | N/A | 2.45 | | 1.03 | | 0.00 | |
| | | **INV** | N/A | 2.43 | 77 | 0.69 | 25 | 0.00 | 0 |
| | | **INV** | N/A | 1.69 | | 0.66 | | 0.00 | |
| Coated Cans | Butyl Rubber Sealing Gaskets (according to the invention) | **UP** | N/A | 2.64 | 99 | 2.73 | 103 | 2.71 | 102 |
| | | **UP** | N/A | 2.67 | | 2.77 | | 2.73 | |
| | | **INV** | N/A | 2.67 | 100 | 2.67 | 100 | 2.64 | 99 |
| | | **INV** | N/A | 2.68 | | 2.71 | | 2.69 | |
| Conventional Aluminium Cans | Butyl Rubber Sealing Gaskets (according to the invention) | **UP** | N/A | 2.67 | 99 | 2.69 | 100 | 2.63 | 98 |
| | | **UP** | N/A | 2.66 | | 2.69 | | 2.64 | |
| | | **INV** | N/A | 2.66 | 100 | 2.70 | 101 | 2.31 | 91 |
| | | **INV** | N/A | 2.70 | | 2.71 | | 2.57 | |
| Anodised Aluminium Cans | Butyl Rubber Sealing Gaskets (according to the invention) | **UP** | N/A | 2.62 | 97 | 1.47 | 56 | 1.01 | 22 |
| | | **UP** | N/A | 2.55 | | 1.51 | | 0.17 | |
| | | **INV** | N/A | 2.65 | 99 | 2.68 | 99 | 1.84 | 72 |
| | | **INV** | N/A | 2.68 | | 2.63 | | 2.01 | |

The experimental results show that the addition of a mineral acid, in particular 0.01% w/w phosphoric acid 15M improves the stability of a tiotropium salt, preferably the bromide salt, both in coated cans and in conventional aluminium cans.

However only in the cans fitted with metering valves having sealing rings and/or gaskets made of a butyl rubber the stability of tiotropium bromide is improved at a pharmaceutical acceptable level up to 3 months at 40°C both in conventional aluminium cans and in cans lined by an inert coating comprising a fluorinated polymeric mixture.

Particularly stable tiotropium solution formulation are obtained when the formulation is filled in coated cans fitted with metering valves having sealing rings and/or gaskets made of a butyl rubber. The stability of the active ingredient is then improved at a pharmaceutical acceptable level up to 6 months at 40°C, which are very drastic storage conditions.

Therefore to improve the stability of tiotropium bromide HFA aerosol solution formulation is not only necessary to add small amounts of an acid but also the selection of the type of the container and of the metering valve and in particular of the type of sealing gaskets which may be in contact with the formulation.

A butyl rubber, a halo-butyl rubber or a vulcanisate of an elastomeric composition of a butyl rubber as described in WO 03/078538 represent the material of choice for sealing gaskets of metered dose valves to reduce the interaction with the aerosol solution formulation which negatively influence the stability of a tiotropium salt.

## Claims

1. A pressurized metered dose inhaler comprising an aerosol can fitted with a metering valve comprising sealing ring/s and/or gasket/s, which is/are in contact with the formulation, made of a butyl or halo-butyl rubber wherein the aerosol can contains a medicinal aerosol solution formulation comprising
a tiotropium salt,
a hydrofluorocarbon propellant,
one or more co-solvents, and
a mineral acid.

2. A pressurized metered dose inhaler of claim 1 wherein the butyl or halo-butyl rubber is a vulcanisate of an elastomeric composition of a butyl or halo-butyl rubber, a cross-linking agent for the butyl or halo-butyl rubber, and an accelerator for the cross-linking agent, in particular the accelerator may include a polysulphide compound derived from a substituted dithiocarbonic acid or derivative thereof.

3. A pressurized metered dose inhaler, according to claims 1 or 2 wherein the tiotropium salt is tiotropium bromide.

4. A pressurized metered dose inhaler, according to anyone of claims 1 to 3 wherein the hydrofluorocarbon propellant is HFA 134a, HFA 227 or a mixture thereof.

5. A pressurized metered dose inhaler, according to anyone of claims 1 to 4 wherein the co-solvent is ethanol.

6. A pressurized metered dose inhaler, according to anyone of claims 1 to 5 wherein the mineral acid is hydrochloric acid.

7. A pressurized metered dose inhaler, according to anyone of claims 1 to 5 wherein the mineral acid is phosphoric acid.

8. A pressurized metered dose inhaler, according to claim 6 wherein hydrochloric acid 0.02 M is contained in an amount between 0.0003 and 0.15% w/w.

9. A pressurized metered dose inhaler, according to claim 7 wherein phosphoric acid 15 M is contained in the medicinal aerosol formulation in an amount between 0.0004 and 0.040% w/w.

10. A pressurized metered dose inhaler, according to anyone of claims 1 to 9 wherein part or all of the internal surfaces of the aerosol can consists of conventional aluminium, anodised aluminium or is lined with an inert organic coating which is a perfluoroalkoxy alkane, a perfluoroalkoxyalkylene, a perfluoroalkylene such as polytetrafluoroethylene, epoxy-phenol resin or fluorinated ethylene-propylene, polyether sulfone or blends thereof.

11. A process for filling a pressurized metered dose inhaler according to any one of claims 1 to 10, comprising:
filling said can with a solution of a tiotropium salt in one or more co-solvent,
adding a pre-determined amount of a mineral acid,
further adding a propellant comprising a hydrofluoroalkane (HFA) to said solution and
crimping with the metering valve.

12. A process for filling a pressurized metered dose inhaler according to any one of claims 1 to 10, comprising:
filling said can with a solution of a tiotropium salt in one or more co-solvent,
adding a pre-determined amount of a mineral acid,
crimping the valve on the can and
adding the propellant through the valve.

13. The use of the pressurised metered dose inhaler according to anyone of claims 1 to 12 for the preparation of a medicament for the prevention and/or treatment of an inflammatory or obstructive airways disease such as asthma or chronic obstructive pulmonary disease (COPD).
